# EUROPEAN PATENT APPLICATION

(11) **EP 1 884 243 A1**
(43) Date of publication of application: **06.02.2008**
(21) Application number: 06009051.1
(22) Date of filing: 02.05.2006
(51) Int. Cl.: A61K 31/551, A61P 3/00

(54) **Use of an antipsychotic drug for the treatment of ALS-associated cachexia**

(71) Applicant: Charité - Universitätsmedizin Berlin, 10117 Berlin (DE)
(72) Inventor: Meyer, Thomas, 12587 Berlin (DE)
(74) Representative: Engelhard, Markus

(57) **Abstract**

The present invention relates to the use of an antipsychotic drug for the treatment of cachexia caused by amyotrophic lateral sclerosis.

## Description

The present invention relates to new uses of an antipsychotic drug.

Amyotrophic lateral sclerosis (ALS) is a neurodegenerative disease with an onset during adulthood. ALS is characterized by a selective degeneration of motor neurons in the brain and the spinal cord. As a consequence of the degeneration of such motor neurons, progressive paralysis of muscles develop in the extremities, in the speech apparatus and in the throat. The etiology and pathogenesis of ALS is not clear. At an average, 3-5 years after onset of ALS, patients die because of respiratory insufficiency. The onset of the disease is between the third and eighth decade, the maximum being in the fifth decade. 2 in 100.000 humans develop the disease. In Germany, approximately 6000 patients are affected. Annually, about 1500 patients die of ALS in the Federal Republic of Germany.

Current treatment of ALS is by means of administration of Riluzole (Rilutek® of Aventis-Sanofi-Synthelabo) which is the only approved pharmacological therapy for ALS. Riluzole is an inhibitor of the presynaptic release of glutamate aiming at neuroprotection. Riluzole has the effect of prolonging an ALS-patient's lifetime by about 1.5-2.5 months. Hence, an effective therapy of ALS is presently not available. Despite its low clinical efficacy, Riluzole is the standard medication and appears to be the only drug that is currently approved for treating ALS. Because of the low clinical efficacy of Riluzole, there is therefore a need in the art for a more efficient treatment of ALS.

In a number of ALS-patients, more specifically in about 16% of patients (Desport et al., Neurology, 1990, 53:1059-1063) to 53% (Mazzini et al., 1995, J. Neurol. 1995; 242:695-698) a process of physical wasting with loss of weight and muscle mass can be observed, also referred to as ALS-associated cachexia. Controlled studies have shown that in 15-50% of ALS-patients, there is a cachexia which, in some studies, may be defined by a decrease of the body mass index (BMI) which in these patients is < 20 kg/m², or by a loss of body weight > 7%. Systematic trials have shown that cachexia is a factor of negative prognosis for ALS-patients, since ALS-patients having a BMI<18.5 kg/m² show a 7.7-fold increased risk of dying in the subsequent 7 months in comparison to ALS-patients without such cachexia ( Desport et al., Neurology, 1999, 53:1059-1063). ALS-associated cachexia is an independent factor of prognosis which shows no correlation to respiratory function, dysphagia or other symptoms of ALS. ALS-associated cachexia therefore appears to be an independent symptom of ALS. Currently, there is no treatment available which is aimed at the reduction of ALS-associated cachexia in ALS-patients. It was therefore an object of the present invention to provide for means that allow to reduce the body weight loss and/or to stabilize or even increase the body mass index in ALS-associated cachexia patients.

The objects of the present invention are solved by the use of an antipsychotic drug for the manufacture of a medicament for the prevention and/or treatment of amyotrophic lateral sclerosis (ALS)-associated cachexia in a patient.

In one embodiment said ALS-associated cachexia is characterized by a weight loss of > 7% in said patient.
Preferably, said ALS-associated cachexia is characterized by a body mass index (BMI) of said patient < 24 kg/m², more preferably, < 22 kg/m², even more preferably, < 20 kg/m², and most preferably < 18.5 kg/m².

In those instances, where said antipsychotic drug is used for the prevention of amyotrophic lateral sclerosis (ALS)-associated cachexia, said prevention is indicated in a patient experiencing a loss of appetite or a loss of body weight > 1 %, or said prevention of ALS-associated cachexia is indicated in a patient having a body mass index < 24 kg/m².

As used herein, the term "loss of appetite" or "loss of body weight" usually refers to a unintended loss of appetite or unintended loss of body weight. Where the term "loss of body weight" is further specified by a percentage, this is usually in relation to the total body weight. Hence, for example a loss of body weight of > 7% means that the patient lost > 7% of his total body weight.

Usually ALS-associated cachexia is observed 1 week to 5 years after the onset of one or several ALS-related symptoms. Therefore, in one preferred embodiment, said ALS-associated cachexia is characterized by a weight loss of > 7% in said patient, wherein said weight loss is observed or occurs after 1 week to 5 years after the onset of at least one ALS-related symptom. In another embodiment, said weight loss of > 7% in said patient occurs or is observed over a period of 1 week to 5 years after the onset of at least one ALS-related symptom. Preferably, said at least one ALS-related symptom is selected from the group comprising paresis, muscle atrophy, muscle weakness, fasciculation, cramp, spasticity, dyspnoea, dysarthria, dysphagia and pseudobulbar effect, such as pathologic laughing and/or crying.

Likewise, where said antipsychotic drug is used for the prevention of amyotrophic lateral sclerosis (ALS)-associated cachexia, where said prevention is indicated in a patient experiencing a loss of body weight > 1%, preferably said loss of body weight > 1% occurs or is observed after 1 week to 5 years after the onset of at least one ALS-related symptom. In another embodiment, said loss of body weight > 1 % occurs or is observed over a period of 1 week to 5 years after the onset of at least one ALS-related symptom. Similar considerations apply to a loss of appetite which may occur or be observed concomitantly with said loss of body weight. Preferably, said at least one ALS-related symptom is as defined above.

In one embodiment said antipsychotic drug is selected from the group comprising phenothiazines, thioxanthines, butyrophenones, diphenylbutylpiperadines, indolones, dibenzepines, benzodiazepines, and thienobenzodiazepines.

In one embodiment said antipsychotic drug is selected from the group comprising olanzapine, promethacine, chlorprothixen, levomepromazine, prothipendyl, perazine, triflupromazine, fluspirilene, pimozide, salpiride, chlorpromazine, fluphenazine, thioridazine, haloperidol, clozapine, risperidone, quetiapine, ziprasidone.

In one embodiment said antipsychotic drug is a typical antipsychotic drug.

In another embodiment said antipsychotic drug is an atypical antipsychotic drug.

In one embodiment said antipsychotic drug is a typical antipsychotic drug and is selected from the group comprising chlorpromazine, fluphenazine, thioridazine and haloperidol.

In another embodiment said antipsychotic drug is an atypical antipsychotic drug and is selected from the group comprising clozapine, risperidone, olanzapine, quetiapine and ziprasidone.

In one preferred embodiment said antipsychotic drug is olanzapine.

In one embodiment said antipsychotic drug is administered at a dose of 1 mg to 1500 mg/day, wherein, preferably, said antipsychotic drug is administered at a dose of 1 mg to 100 mg/day.

If the antipsychotic drug is olanzapine, it is preferred that said antipsychotic drug is administered at a dose of 1 mg to 40 mg/day, preferably 1 mg - 30 mg/day, and more preferably 1 mg - 20 mg/day.

Preferably, said antipsychotic drug is administered in combination with at least one other drug used for the treatment of amyotrophic lateral sclerosis and/or its symptoms. More preferably, said at least one other drug used for the treatment of amyotrophic lateral sclerosis and/or its symptoms is selected from the group comprising riluzole, citalopram and other serotonin reuptake inhibitors, mirtazepin, reboxetin, fluoxetin, fluvoxamine, amitriptylin, lorazepam, diazepam, baclofen, tizanidin, scopolamine, and atropine.

The term "said antipsychotic drug is administered in combination with at least one other drug", as used herein is meant to signify that the two drugs are administered together via the same route or at the same time or both, but this term is also meant to encompass such scenarios where one drug is administered after the other or via different routes or a combination thereof. The two drugs may be formulated together in one composition, or they may be in different formulations or administration units.

In one embodiment said treatment results in an attenuation of body weight loss and/or an increase in body weight and/or a stabilization or increase in body mass index and/or a mobilization of residual motor function and/or an improvement in life quality in said patient.

Preferably, said patient is a patient diagnosed with ALS or a patient suspected of developing ALS.

Preferably, said patient is a human being.

The present disclosure also relates to methods of treatment and/or prevention of ALS-associated cachexia in a patient, using an antipsychotic drug, wherein the drug, its administration, the patient and the treatment is as defined in any of the foregoing embodiments and/or the description hereafter.
The term "antipsychotic drug" as used herein is meant to refer to a drug that is used to treat psychotic symptoms, such as schizophrenia, psychoses, obsessive-compulsive disorders, bipolar disorders etc. Chemically, most antipsychotic drugs are phenothiazines, thioxanthines, butyrophenones, diphenylbutylpiperadines, indolones, dibenzepines, benzodiazepines, and thienobenzodiazepines. The term "typical antipsychotic drug" refers to an antipsychotic drug which has been developed and/or approved as antipsychotic drug between 1950 and 1970. This term is also synonymously used herein with other terms used in the literature, such as "older" or "conventional" antipsychotic drug. Examples of such "typical" antipsychotic drugs are chlorpromazine, released in Europe in 1952, fluphenazine, released in the US in 1959, thioridazine, released in the US in 1959, and haloperidol, released in the US in 1967. In contrast thereto an "atypical" antipsychotic drug, as used herein, refers to an antipsychotic drug which has been released after 1970 in Europe or the US and/or which is believed to show fewer side effects. Examples of such "atypical" antipsychotic drugs are clozapine, risperidone, olanzapine, quetiapine and ziprasidone, all of which have been released in Europe or the US after 1970 (clozapine 1975, risperidone 1994, olanzapine 1997, quetiapine 1997, and ziprasidone 2001).

Sometimes, "antipsychotic drugs" are also referred to as "neuroleptic drugs" or "neuroleptics".

The term " amyotrophic lateral sclerosis", as used herein is meant to refer to a progressive neurodegenerative disorder which affects the upper and lower motor neuron or the lower motor neuron alone or the upper motor neuron alone.
The term "ALS-associated cachexia" as used herein is a loss of body weight which occurs prior to or concomitant with or after a clinical manifestation of amyotrophic lateral sclerosis.
A clinical manifestation of ALS is preferably the occurrence of one or several ALS-related symptoms, as defined further above.

Such "ALS-associated cachexia" is characterized either by a body weight loss of >7% of a patient. and/or a body mass index (BMI) < 24 kg/m², preferably < 22 kg/m², more preferably < 20 kg/m² and most preferably < 18.5 kg/m². In one embodiment said loss of body weight or said BMI-value may be associated with a loss of appetite.

The term "body mass index (BMI)" as used herein is a measure of the weight of a person scaled according to height. Roughly speaking for adult individuals, a BMI of less than 20 implies under weight, over 25 is overweight, and over 30 is obese. The BMI is calculated by taking the weight of the individual in kg and dividing by the square of the height in meters.

The inventors have surprisingly found that using an antipsychotic drug, such as olanzapine, in the treatment or prevention of ALS-associated cachexia leads to a reduction and/or prevention of body weight loss and/or an increase or stabilization of the body mass index and/or a weight gain and/or a mobilization of residual motor function and generally an improvement in the quality of life of the affected individuals. As a consequence of these results, the prognosis of such patients is improved also with respect to the general course of ALS.

In those instances, wherein said antipsychotic drug is administered for the prevention of ALS-associated cachexia, this is usually indicated in patients with a loss of appetite or loss of body weight > 1% or it is indicated in a patient having a body mass index (BMI) < 24 kg/m².

The antipsychotic drugs may be administered in a composition, preferably a composition suitable for systemic administration. With respect to olanzapine which is also the antipsychotic drug that was used in the experiments performed by the inventor, compositions suitable for internal administration contained from about 0.5 mg to about 50 mg olanzapine per unit.

Typical compositions include olanzapine or a pharmaceutically acceptable acid addition salt thereof, associated with a pharmaceutically acceptable excipient which may be a carrier, or a diluent or be diluted by a carrier, or enclosed within a carrier which can be in the form of a capsule, sachet, paper, or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone. The formulations may also include wetting agents, emulsifying and suspending agents, preserving agents, sweetening agents, or flavoring agents. The formulations of the invention may be formulated so as to provide quick, sustained, or delayed release of the active ingredient after administration to the patient by employing procedures well known in the art.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

Generally, olanzapine is dispensed in unit form comprising from about 1 mg to about 50 mg in a pharmaceutically acceptable carrier per unit dosage.

Most preferably, the solid oral formulation is contained in packaging materials which protect the formulation from moisture and light. For example, suitable packaging materials include amber colored high density polyethylene bottles, amber colored glass bottles, and other containers made of a material which inhibits the passage of light. Most preferably, the packaging will include a desiccant pack. The container may be sealed with an aluminum foil blister to provide the desired protection and maintain product stability.

The compositions of this invention may be suitable for administration to an animal. Such animals include both domestic animals, for example livestock, laboratory animals, and household pets, and non-domestic animals such as wildlife. More preferably, the animal is a vertebrate. Most preferably, a compound of this invention shall be administered to a mammal. It is especially preferred that the animal is a domestic mammal or a human. The most preferred mammal is a human. For such purposes, a compound of this invention may e. g. be administered as a feed additive.

Antipsychotic drugs have so far not been used in the prevention and/or treatment of ALS-associated cachexia. Antipsychotic drugs are believed to have a number of side effects, such as extrapyramidal reactions, including acute dystonias, akathisia, rigidity and tremor, tardive dyskinesia, tachycardia, hypotension, impotence, lethargy, seizures, hyperprolactinaemia and agranulocytosis. Without wishing to be bound by any theory, it is believed that the antipsychotic drugs, in accordance with the present invention, lead to a change in composition of the body by modulating neuronal systems. It may be that for example olanzapine shifts the individual's metabolism toward anabolic processes.

The positive effect of such antipsychotic drugs appears to be restricted to those patients showing ALS-associated cachexia whereas those patients having no such cachexia show no improvement
In the following reference is made to the following examples which are meant to illustrate, not to limit the present invention.

### Examples

### Study

A 60 years old male patient is reported, who at the age of 54 years developed a slowly-progressive motor neuron syndrome. The patient fulfilled the criteria for definite ALS. The disease began with atrophy and weakness of the small muscles of the left hand followed by weakness of the right distal arm. In the course of 2 years after onset he experienced slowly progressive atrophy and flaccid paresis of the upper limbs with only residual functions in handling articles. After 4 years he developed a gait disorder which progressed to the point were he required a walking-aid. At this time he showed clinical signs of low grade hypoventilation. The patient was without dysphagia. Neuropsychological assessment revealed no significant impairment of memory, attention and executive function.

During the course of 10 months, the patient developed an unintended weight loss of 12 kg of body weight resulting in fatigue and reduced mobility. The patient suffered from diminished appetite. The body mass index (BMI) was 17.2 kg/m² and fulfilled the diagnostic criteria of cachexia. For the pharmacological intervention of ALS-related cachexia the medication with olanzapine was initiated. Olanzapine was given orally in the dosage of 5 mg per day. Olanzapine was administered twice daily using one tablet of olanzapine 2.5 mg each in the morning and in the evening. The medication resulted in a weight gain of 6 kg within the course of 8 weeks. The BMI increased to 18,8 kg/m². The nutritional state and the general strength improved resulting in increased mobility of the patient. Residual motor functions were mobilized. The walking distance reached the level before the onset of cachexia. The ALS function rating scale (ALS-FRS) was stabilized and did not decrease. The patient described an improved quality of life. At the time of this report, the olanzapine treatment was ongoing. Olanzapine medication for the treatment of ALS cachexia was not associated with adverse events.

### Study 2

A 69 years old female patient is reported, who at the age of 64 years developed a slowly-progressive motor neuron syndrome. The patient fulfilled the criteria for ALS. The disease began with atrophy and weakness of the small muscles of the right hand followed by weakness of the proximal muscles of right arm and the opposite upper limb. In the course of 4 years after onset she experienced progressive atrophy and flaccid paresis of the lower limbs resulting in a gait disorder. At the time of this report she showed clinical signs of low grade hypoventilation. She did not need ventilatory support. The patient was without dysphagia. Neuropsychological assessment revealed no significant impairment of memory, attention and executive function.

During the course of 12 months, the patient developed an unintended weight loss of 8.5 kg of body weight. The weight loss was associated with fatigue. The patient suffered from diminished appetite. The body mass index (BMI) reduced from 23.3 kg/ m² to 20.5 kg/m². At the same time the ALS functional rating scale (ALS-FRS revised) changed from 43 to 39 units. Most of the wasting occurred within 6 month before intervention (loss of 6.5 kg of body weight; total body weight of 61.5 kg). For the pharmacological intervention of ALS-related cachexia the medication with olanzapine was initiated. Olanzapine was given orally in the dosage of 5 mg per day. Olanzapine was administered once daily using one tablet of olanzapine 5 mg in the evening. The medication attenuated ALS-related cachexia and wasting. After 2 month of olanzapine treatment the body weight was stabilized (61.0 kg). The loss of BMI was also attenuated (20.3 kg/m²). The nutritional state and the general strength improved resulting in increased mobility of the patient. Residual motor functions were mobilized. The patient described an improved quality of life. At the time of this report, the olanzapine dosage was increased to 10 mg per day. Olanzapine was administered twice daily using one tablet of olanzapine 5 mg each in the morning and in the evening. Olanzapine medication for the treatment of ALS cachexia was not associated with adverse events. The olanzapine treatment was ongoing. Based on reports on olanzapine-related weight gain in patients with psychosis, the target dose of olanzapine for the treatment of ALS-cachexia is 20 mg per day. In the case of persistent ALS-related weight loss, the treatment with 30 mg olanzapine per day is recommended.

### Study 3

A 51 years old female patient is reported, who at the age of 48 years developed a slowly-progressive motor neuron syndrome. The patient fulfilled the criteria for ALS. The disease began with atrophy and weakness of the proximal left arm followed by weakness of the distal muscles of left upper limb. In the course of 2 years after onset she experienced progressive atrophy and flaccid paresis of the opposite limb with only residual functions in handling articles. After 3 years the disorder progressed to the point that was complete loss of manual functions. The patient developed hypoventilation resulting in ventilatory support using non-invasive intermittent positive pressure ventilation (NIPPV). The patient was without dysphagia. Neuropsychological assessment revealed no significant impairment of memory, attention and executive function.

During the course of 9 months, the patient developed an unintended weight loss of 14.5 kg of body weight. The weight loss was associated with fatigue. The patient suffered from diminished appetite. The body mass index (BMI) reduced from 23.0 kg/ m² to 17.5 kg/m². Most of the wasting occurred within 5 month before intervention (loss of 8.5 kg of body weight; total body weight reduced from 55 kg to 46.5 kg). For the pharmacological intervention of ALS-related cachexia the medication with olanzapine was initiated. Olanzapine was given orally in the dosage of 5 mg per day. Olanzapine was administered once daily using one tablet of olanzapine 5 mg in the evening. After 4 weeks of treatment, olanzapine dosage was increased to 10 mg per day. Olanzapine was administered twice daily using one tablet of olanzapine 5 mg each in the morning and in the evening. The medication attenuated ALS-related cachexia and wasting. After 2 month of olanzapine treatment the body weight was stabilized (45.5 kg). The loss of BMI was also attenuated (17.1 kg/m²). The nutritional state and the general strength improved. Olanzapine medication for the treatment of ALS cachexia was not associated with adverse events. The olanzapine treatment was ongoing. Based on reports on olanzapine-related weight gain in patients with psychosis, the target dose of olanzapine for the treatment of ALS-cachexia is 20 mg per day. In the case of persistent ALS-related weight loss, the treatment with 30 mg olanzapine per day is recommended.

The features of the present invention disclosed in the specification, the claims and/or in the accompanying drawings, may, both separately, and in any combination thereof, be material for realising the invention in various forms thereof.

## Claims

1. Use of an antipsychotic drug for the manufacture of a medicament for the prevention and/or treatment of amyotrophic lateral sclerosis (ALS)-associated cachexia in a patient.

2. Use according to claim 1, wherein said ALS-associated cachexia is **characterized by** a weight loss of > 7% in said patient.

3. Use according to claim 1 or claim 2, wherein said ALS-associated cachexia is **characterized by** a body mass index (BMI) of said patient < 24 kg/m².

4. Use according to claim 3, wherein said ALS-associated cachexia is **characterized by** a body mass index of said patient < 22 kg/m².

5. Use according to claim 4, wherein said ALS-associated cachexia is **characterized by** a body mass index of said patient < 20 kg/m².

6. Use according to claim 5, wherein said ALS-associated cachexia is **characterized by** a body mass index of said patient < 18.5 kg/m².

7. Use according to any of the foregoing claims, wherein said prevention of ALS-associated cachexia is indicated in a patient having a loss of appetite or a loss of body weight > 1%, or said prevention of ALS-associated cachexia is indicated in a patient having a body mass index < 24 kg/m².

8. Use according to any of the foregoing claims, wherein said antipsychotic drug is selected from the group comprising phenothiazines, thioxanthines, butyrophenones, diphenylbutylpiperadines, indolones, dibenzepines, benzodiazepines, and thienobenzodiazepines.

9. Use according to any of the foregoing claims, wherein said antipsychotic drug is selected from the group comprising promethacine, chlorprothixen, levomepromazine, prothipendyl, perazine, triflupromazine, fluspirilene, pimozide, salpiride, chlorpromazine, fluphenazine, thioridazine, haloperidol, clozapine, risperidone, quetiapine, ziprasidone, and olanzapine.

10. Use according to any of the claims 1-7, wherein said antipsychotic drug is a typical antipsychotic drug.

11. Use according to any of claims 1-7, wherein said antipsychotic drug is an atypical antipsychotic drug.

12. Use according to any of claims 8 and 10, wherein said antipsychotic drug is selected from the group comprising chlorpromazine, fluphenazine, thioridazine and haloperidol.

13. Use according to any of claims 8 and 11, wherein said antipsychotic drug is selected from the group comprising clozapine, risperidone, olanzapine, quetiapine and ziprasidone.

14. Use according to claim 13, wherein said antipsychotic drug is olanzapine.

15. Use according to any of the foregoing claims, wherein said antipsychotic drug is administered at a dose of 1 mg to 1500 mg/day.

16. Use according to claim 15, wherein said antipsychotic drug is administered at a dose of 1 mg to 100 mg/day.

17. Use according to any of claims 14 and 16, wherein said antipsychotic drug is administered at a dose of 1 mg to 40 mg/day, preferably 1 mg - 30 mg/day, and more preferably 1 mg - 20 mg/day.

18. Use according to any of the foregoing claims, wherein said antipsychotic drug is administered in combination with at least one other drug used for the treatment of amyotrophic lateral sclerosis and/or its symptoms.

19. Use according to claim 18, wherein said at least one other drug used for the treatment of amyotrophic lateral sclerosis and/or its symptoms is selected from the group comprising riluzole, citalopram and other serotonin reuptake inhibitors, mirtazepin, reboxetin, fluoxetin, fluvoxamine, amitriptylin, lorazepam, diazepam, baclofen, tizanidin, scopolamine, and atropine.

20. Use according to any of the foregoing claims, wherein said treatment results in an attenuation of body weight loss and/or an increase in body weight and/or a stabilization or increase in body mass index and/or a mobilization of residual motor function and/or an improvement in life quality in said patient.

21. Use according to any of the foregoing claims, wherein said patient is a patient diagnosed with ALS or a patient suspected of developing ALS.

22. Use according to any of the foregoing claims wherein said patient is a human being.
